# EUROPÄISCHE PATENTSCHRIFT

(11) **EP 0 594 580 B1**
(45) Veröffentlichungstag und Bekanntmachung des Hinweises auf die Patenterteilung: **20.03.1996**
(21) Anmeldenummer: 91902688.0
(22) Anmeldetag: 15.01.1991
(51) Int. Cl.: C07C 69/587, C07C 67/347

(54) **OLEFINISCH UNGESÄTTIGTE ADDUKTE VON PROPYLEN AN MEHRFACH UNGESÄTTIGTE FETTSÄUREN BZW. FETTSÄUREESTER, VERFAHREN ZU IHRER HERSTELLUNG UND IHRE VERWENDUNG**
OLEFINICALLY UNSATURATED ADDUCTS OF PROPYLENE ON POLYUNSATURATED FATTY ACIDS OR FATTY ACID ESTERS, PROCESS FOR PRODUCING THEM AND THEIR USE
PRODUITS D'ADDITION OLEFINIQUEMENT INSATURES DU PROPYLENE SUR DES ACIDES GRAS MULTI-INSATURES OU SUR LEURS ESTERS, LEUR PROCEDE DE FABRICATION ET LEUR UTILISATION

(30) Priorität: 24.01.1990 DE 4002008
(43) Veröffentlichungstag der Anmeldung: 04.05.1994
(73) Patentinhaber: Henkel Kommanditgesellschaft auf Aktien, D-40191 Düsseldorf (DE)
(72) Erfinder: LAUFENBERG, Alfred, D-4047 Dormagen 5 (DE); BEHR, Arno, D-4000 Düsseldorf 13 (DE)
(86) Internationale Anmeldenummer: EP9100052
(87) Internationale Veröffentlichungsnummer: WO9111427

(56) Entgegenhaltungen:
- EP-A- 0 206 367
- US-A- 3 966 798
- US-A- 4 318 860

## Beschreibung

Die Erfindung betrifft olefinisch ungesättigte Addukte von Propylen an mehrfach ungesättigte Fettsäuren mit 18 bis 22 Kohlenstoffatomen bzw. an Ester derselben mit C₁-C₃₆-Alkanolen in molaren Verhältnissen von Propylen zu den Fettsäuren bzw. Fettsäureestern im Bereich von 1 : 1 bis 2 : 1, erhältlich durch Umsetzung der Fettsäuren bzw. Fettsäureester mit Propylen bei erhöhter Temperatur und erhöhtem Druck in Gegenwart von Verbindungen von Übergangsmetallen aus der von Ru, Rh, Pd, Ir und Pt gebildeten Gruppe als Katalysatoren.

In der Alkylkette verzweigte Fettsäuren vom Typ der Guerbetsäuren, erhältlich durch Guerbetisierung der entsprechenden Fettalkohole und Oxidation der Guerbetalkohole zu den entsprechenden Säuren, sind technologisch interessante Zwischenprodukte, weil sie bzw. ihre Alkylester, im Vergleich zu den entsprechenden unverzweigten Isomeren, deutlich erniedrigte Stockpunkte aufweisen. Die Herstellung von Guerbetsäuren ist jedoch technologisch aufwendig und nur mit unbefriedigenden Ausbeuten durchführbar. Es hat daher nicht an Versuchen gefehlt, ausgehend von Fettsäuren bzw. Estern derselben entsprechende Fettsäurederivate herzustellen, die in der Alkylkette Verzweigungen aufweisen. Ein typisches Beispiel hierfür ist die schichtsilikatkatalysierte Fettsäuredimerisation, bei der jedoch auch erhebliche Mengen an trimeren Fettsäuren sowie an methylverzweigten Fettsäuren, sogenannten Isofettsäuren, gebildet werden. Ein weiteres, wenn auch aufwendiges Verfahren liefert aus Konjuenfettsäuren in der trans,trans-Form mit aktivierten Dienophilen unter den Bedingungen einer Diels-Alder-Reaktion verzweigte Fettsäurederivate; so läßt sich z.B. auf diesem Wege aus Linolsäure und Acrylsäure eine verzweigte C₂₁-Dicarbonsäure herstellen, vgl. US-B 3,734,859, US-B 3,753,968, DE-B 2 253 930. Weitere, verzweigte Fettsäurederivate sind durch thermische oder säurekatalysierte Addition von aktivierten Enophilen an ungesättigte Fettsäurederivate erhalten worden. So läßt sich z.B. Maleinsäureanhydrid unter Säurekatalyse mit bis zu 70 % Ausbeute an Ölsäure addieren, vgl. Fat. Sci. Technol., 1, 1 (1988). Bei den vorstehend genannten Reaktionsprodukten erweist sich die Anwesenheit von mehr als einer Carboxylgruppe jedoch häufig als störend.

Schließlich hat man auch versucht, gesättigte Kohlenwasserstoffe durch thermisch initiierte radikalische Addition von gesättigten Kohlenwasserstoffen an Fettsäuren anzulagern; die Addition von Cyclohexan an Ölsäuremethylester führt bei 340°C und 200 bar mit 70%-iger Selektivität, jedoch mit einer Ausbeute von lediglich 2,8 %, zu alkylverzweigten Fettsäuren, vgl. J.O. Metzger, et al., Fat. Sci. Technol. 1 (1989), 18.

Aus der Europäischen Patentanmeldung EP-A 0010807 sind olefinisch ungesättigte Addukte von einfach verzweigten ungesättigten Fettsäuren bzw. deren Estern bekannt, wobei die Verzweigungen Propenyl- oder i-Hexenylreste darstellen.

Die Erfindung ist auf die Bereitstellung olefinisch ungesättigter Addukte von Propylen an mehrfach ungesättigte Fettsäuren der eingangs genannten Art gerichtet, die auf einfache Weise und mit guten Ausbeuten hergestellt werden können. Die erfindungsgemäß bereitgestellten Verbindungen sind neue Produkte, die sich z.B. von in der Natur vorkommenden ethylverzweigten Fettsäuren mit insgesamt 12 bis 18 Kohlenstoffatomen, beschrieben in A. Smith et al, Biomed. Mass Spectrom., 6 (8), 347-349, bereits durch ihre Kettenlänge unterscheiden.

Als Ausgangsprodukte zur Herstellung der olefinisch ungesättigten Addukte der Erfindung eignen sich ungesättigte Fettsäuren mit 18 bis 22 Kohlenstoffatomen und mehr als einer olefinischen Doppelbindung wie Linolsäure, isomerisierte Linolsäure mit konjuierten Doppelbindungen (sogenannte C18:2-Konjuenfettsäure), Linolensäure, Arachidonsäure, Docosadiensäure, Docosahexaensäure und Eicosapentaensäure, die in Form ihrer technischen Gemische mit anderen Fettsäuren nachwachsenden natürlichen Rohstoffen, z.B. aus Sonnenblumenöl, Tallöl oder Fischöl, erhalten werden können. Diese mehrfach ungesättigten Fettsäuren werden, wie in der Fettchemie üblich, im allgemeinen nicht in Form ihrer reinen Verbindungen, sondern in Form ihrer technischen Gemische zur Herstellung der Addukte der Erfindung eingesetzt. Die vorgenannten Fettsäuren werden bevorzugt nicht nur als solche, sondern auch in Form ihrer Ester mit C₁-C₃₆-Alkanolen, insbesondere mit C₁-C₄-Alkanolen, eingesetzt. Typische Beispiele für derartige Alkanole zur Bildung von Estern mit den vorgenannten Fettsäuren sind Methanol, Ethanol, Propanol, Butanol, Pentanol, Hexanol, Octanol, Decanol, Dodecanol, Tetradecanol, Hexadecanol, Octadecanol sowie höhere Fettalkohole bzw. Fettalkoholderivate mit bis zu 36 Kohlenstoffatomen, z.B.
C₃₆-Guerbetalkohole.

Die vorgenannten mehrfach ungesättigten Fettsäuren bzw. Fettsäureester lagern erfindungsgemäß bei erhöhter Temperatur und erhöhtem Druck in Gegenwart von Verbindungen von Übergangsmetallen aus der von Ru, Rh, Pd, Ir und Pt gebildeten Gruppe Propylen an.

Typische Beispiele für erfindungsgemäß einsetzbare Katalysatoren sind die folgenden:
RhCl₃.3H₂O
RhBr₃.3H₂O
[(C₂H₄)₂RhCl]₂
Rh(NO₃)₃.2H₂O
Rh(OOCCH₃)₂.2H₂O
Rh(acetylacetonat)₃
RhF₃.6H₂O
RhJ₃
Rh(CN)₃.3H₂O
Rh₂(SO₄)₃
Rh₂(CO₃)₃
[(1.5-Cyclooctadien)RhCl]₂
[(C₂H₄)₂Rh(acetylacetonat)]
[(1.3-Butadien)RhCl]₂
Cyclopentadienyl-Olefin-Komplexe wie [(n-C₅H₅)Rh(C₂H₄)₂].

Sofern die erfindungsgemäß einsetzbaren Katalysatoren in wasserfreier Form vorliegen, kann es zweckmäßig sein, dem Reaktionsgemisch eine geringe Menge von Wasser zuzusetzen.

Die im Rahmen der Erfindung einsetzbaren Katalysatoren sind als solche für die Anlagerung von Ethylen an Alkadiene bekannt, vgl. US-B 3,636,122; M. Bochmann et al., Journal of Molecular Catalysis, 22 (1984) 363-365; G. Wilkinson (Ed.), Comprehensive Organometallic Chemistry, Seite 414-429, Pergamon Press (1982); A.C.L. Su, Advances in Organometallic Chemistry, Vol. 17, Seite 271-283; wobei diese Veröffentlichungen, auf deren Inhalt im übrigen ausdrücklich Bezug genommen wird, sich jedoch nicht mit der Anlagerung von Alkenen an Fettsäuren bzw. Fettsäurederivate oder andere Fettstoffe befassen.

Weitere, im Verfahren der Erfindung einsetzbare Katalysatoren sind z.B.
PdCl₂
PtCl₂
IrCl₃
OsCl₃
Ru(acetylacetonat)₃.

Mit den erfindungsgemäß einsetzbaren Katalysatoren entstehen im allgemeinen Gemische von 1:1- und 2:1-Addukten von Propylen an die Fettsäuren bzw. Fettsäureester. Durch Veränderung der Reaktionsbedingungen wie Druck, Temperatur und Reaktionszeit lassen sich jedoch die Anteile an den verschiedenen Addukten variieren. Setzt man dem Reaktionsgemisch jedoch neben den Katalysatoren geeignete Phosphin- oder Phosphitliganden, z.B.
P(C₄H₉)₃
P(OC₄H₉)₃
P(C₆H₅)₃
P(OC₆H₅)₃

oder andere, aus dem zuletzt diskutierten Stand der Technik sowie der DE-B 20 16 133 bekannte Liganden zu, kann man unter Umständen gezielt die Zusammensetzung der Adduktgemische beeinflussen. Ähnliche Effekte lassen sich zum Teil dadurch erreichen, daß man dem Reaktionssystem Promotoren wie LiCl, FeCl₃ oder AgBF₄ zusetzt, die als solche ebenfalls aus dem zuletzt genannten Stand der Technik bekannt sind.

Die Struktur der erfindungsgemäßen olefinisch ungesättigten Addukte ist nicht einheitlich. Im Falle der Linolsäure (bzw. der von dieser abgeleiteten C₁₈-Konjuenfettsäure) konnte gezeigt werden, daß die Anlagerung des ersten Propylenmoleküls zwischen den Positionen 9 und 12 der Kohlenstoffkette der Linolsäure erfolgt, wobei das 1:1-Addukt die gleiche Anzahl von Doppelbindungen wie die als Ausgangsmaterial eingesetzte Fettsäure aufweist. Die Stellung der Doppelbindungen ist jedoch nicht einheitlich. Die Doppelbindungen sind in keinem Fall mehr als 4 Kohlenstoffatome von der Verzweigung entfernt und stehen grundsätzlich in alpha,delta- oder in alpha,gamma-Stellung zueinander. Das zweite Propylenmolekül wird dann an eine in der Verzweigung befindliche Doppelbindung angelagert. Es ist zu vermuten, daß die erfindungsgemäß erhaltenen 1:1- und 2:1-Addukte mindestens teilweise die auf der folgenden Seite wiedergegebene Struktur aufweisen.

Gemäß einer vorteilhaften Ausführungsform der Erfindung weisen die gegebenenfalls in Form ihrer Ester eingesetzten mehrfach ungesättigten Fettsäuren 2 bis 5, insbesondere 2 bis 3 olefinische Doppelbindungen auf.

Gemäß einer weiteren vorteilhaften Ausführungsform der Erfindung erhält man die Addukte bei einem Propylendruck im Bereich von 5 bis 40 bar und einer Temperatur im Bereich von 50 bis 140°C, wobei man gegebenenfalls in Anwesenheit inerter organischer Lösemittel wie Hexan, Chloroform oder dergleichen, arbeitet.

Gemäß einer weiteren vorteilhaften Ausführungsform der Erfindung verwendet man die Katalysatoren in einer Menge im Bereich von 0,02 bis 2 Mol-%, bezogen auf Fettsäuren bzw. Fettsäureester.

Vorteilhafterweise verwendet man als Katalysatoren Rhodiumverbindungen, wobei wiederum Rhodiumverbindungen aus der von RhCl₃ und RhBr₃ (einschließlich Hydraten derselben) sowie [(C₂H₄)₂RhCl]₂ gebildeten Gruppe als Katalysatoren bevorzugt sind.

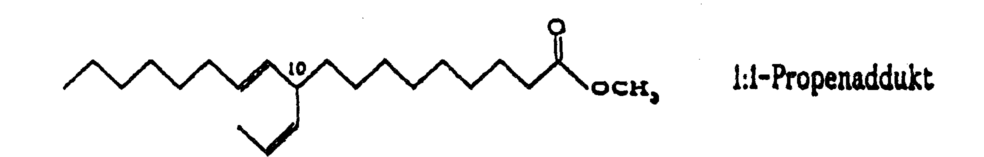

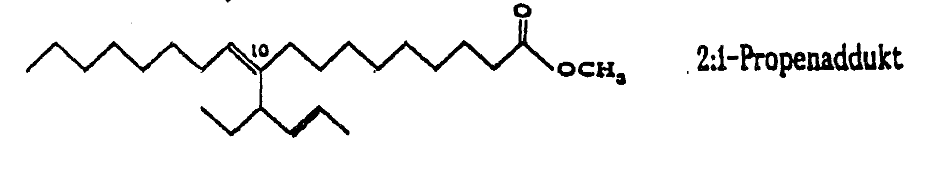

Die Erfindung betrifft weiterhin ein Verfahren zur Herstellung von olefinisch ungesättigten Addukten von Propylen an mehrfach ungesättigte Fettsäuren mit 18 bis 22 Kohlenstoffatomen bzw. an Ester derselben mit C₁-C₃₆-Alkanolen in molaren Verhältnissen von Propylen zu den Fettsäuren bzw. Fettsäureestern im Bereich von 1:1 bis 2:1 mit den oben erläuterten Merkmalen.

Die olefinisch ungesättigten Addukte der Erfindung sind als Ausgangsprodukte für die Herstellung gesättigter, verzweigter Fettsäuren mit 21 bis 28 Kohlenstoffatomen bzw. Ester derselben mit C₁-C₃₆-Alkanolen, die z.B. in kosmetischen Formulierungen und insbesondere in Schmiermitteln eingesetzt werden können, geeignet.

Die Erfindung wird im folgenden anhand von bevorzugten Ausführungsbeispielen näher erläutert.

### Beispiel 1.

8,2 g eines technischen Fettsäuregemisches mit einem Gehalt von 67,8 Gew.-% Linolsäuremethylester (19 mmol), 100 mg RhCl₃.3H₂O und 10 ml Chloroform wurden 20 h bei 100°C in Gegenwart von überschüssigem Propen in einem 75 ml-Autoklaven umgesetzt. Man erhielt 27,6 % (gaschromatografisch ermittelt) an 1:1-Addukten, bezogen auf Linolsäuremethylester.

### Beispiel 2.

8,2 g einer technischen Fettsäure, enthaltend 56,0 % C18:2-Konjuensäuren, 100 mg RhCl₃.3H₂O und 10 ml Hexan wurden 20 h bei 100°C in Gegenwart eines Propylenüberschusses in einem 75 ml-Autoklaven umgesetzt. Man erhielt 70,5 % 1:1-Addukte, bezogen auf Konjuenfettsäure.

### Beispiel 3.

Ein technisches Fettsäuremethylestergemisch mit einem Gehalt von 62,4 % an konjugierten C18:2-Fettsäuremethylestern, 100 mg RhCl₃.3H₂O und 10 ml Hexan wurde 20 h bei 100°C in Gegenwart eines Propylenüberschusses in einem 75 ml-Autoklaven erhitzt. Man erhielt 58,5 % 1:1-Addukte, bezogen auf konjugierte Fettsäuremethylester.

### Beispiel 4.

300 g eines Fettsäuremethylesters gemäß Beispiel 3, 3,0 g RhCl₃.3H₂O und 350 ml Hexan wurden 20 h bei 100°C in Gegenwart eines Propylenüberschusses in einem 1 1-Rührautoklaven mit Turbinenrührer umgesetzt. Es wurden 98,2 % 1:1-Addukte, bezogen auf konjugierte Fettsäuremethylester, erhalten.

### Beispiel 5.

301 g eines Fettsäuremethylestergemisches, enthaltend 60,3 % C18:2-Konjuenmethylester (197 g; 0,76 mol), 6,2 % Linolsäuremethylester und 24,4 % Ölsäuremethylester, 881,6 mg (3,35 mmol) RhCl₃.3H₂O (263,5 g/mol) und 350 ml Hexan wurden in einem 1 1-Rührautoklaven mit Turbinenrührer gegeben. Anschließen wurden ca. 100 g Propen einkondensiert. Das Reaktionsgemisch wurde 20 h bei 100°C gerührt. Man erhielt 91,9 %, bezogen auf Konjuenfettsäureester, an Propylenaddukten, davon 85,2 % 1:1-Addukte und 6,7 % 2:1-Addukte.

## Patentansprüche

1. Olefinisch ungesättigte Addukte von Propylen an mehrfach ungesättigte Fettsäuren mit 18 bis 22 Kohlenstoffatomen bzw. an Ester derselben mit C₁-C₃₆-Alkanolen in molaren Verhältnissen von Propylen zu den Fettsäuren bzw. Fettsäureestern von 1 : 1 bis 2 : 1, erhältlich durch Umsetzung der Fettsäuren bzw. Fettsäureester mit Propylen bei erhöhter Temperatur und erhöhtem Druck in Gegenwart von Verbindungen von Übergangsmetallen aus der von Ru, Rh, Pd, Ir und Pt gebildeten Gruppe als Katalysatoren.

2. Olefinisch ungesättigte Addukte nach Anspruch 1, dadurch gekennzeichnet, daß die mehrfach ungesättigten Fettsäuren 2 bis 5, insbesondere 2 bis 3 olefinische Doppelbindungen aufweisen.

3. Olefinisch ungesättigte Addukte nach Anspruch 1 oder 2, dadurch gekennzeichnet, daß man die Addukte bei einem Propylendruck im Bereich von 5 bis 40 bar und einer Temperatur im Bereich von 50 bis 140°C, gegebenenfalls in Anwesenheit inerter organischer Lösemittel, herstellt.

4. Olefinisch ungesättigte Addukte nach mindestens einem der Ansprüche 1 bis 3, dadurch gekennzeichnet, daß man die Katalysatoren in einer Menge im Bereich von 0,02 bis 2 Mol-%, bezogen auf Fettsäuren bzw. Fettsäureester, verwendet.

5. Olefinisch ungesättigte Addukte nach mindestens einem der Ansprüche 1 bis 4, dadurch gekennzeichnet, daß man als Katalysatoren Rhodiumverbindungen verwendet.

6. Olefinisch ungesättigte Addukte nach mindestens einem der Ansprüche 1 bis 5, dadurch gekennzeichnet, daß man Rhodiumverbindungen aus der von RhCl₃ und RhBr₃ (einschließlich Hydraten derselben) sowie [(C₂H₄)₂RhCl]₂ gebildeten Gruppe als Katalysatoren verwendet.

7. Verfahren zur Herstellung olefinisch ungesättigter Addukte von Propylen an mehrfach ungesättigte Fettsäuren mit 18 bis 22 Kohlenstoffatomen bzw. an Ester derselben mit C₁-C₃₆-Alkanolen in molaren Verhältnissen von Propylen zu den Fettsäuren bzw. Fettsäureestern von 1 : 1, gekennzeichnet durch eine Umsetzung der Fettsäuren bzw. Fettsäureester mit Propylen bei erhöhter Temperatur und erhöhtem Druck in Gegenwart von Verbindungen von Übergangsmetallen aus der von Ru, Rh, Pd, Ir und Pt gebildeten Gruppe als Katalysatoren.

8. Verfahren zur Herstellung der olefinisch ungesättigten Addukte nach Anspruch 7, dadurch gekennzeichnet, daß man mehrfach ungesättigte Fettsäuren verwendet, die 2 bis 5, insbesondere 2 bis 3 olefinische Doppelbindungen aufweisen.

9. Verfahren nach Anspruch 7 oder 8, dadurch gekennzeichnet, daß man die Addukte bei einem Propylendruck im Bereich von 5 bis 40 bar und einer Temperatur im Bereich von 50 bis 140°C, gegebenenfalls in Anwesenheit inerter organischer Lösemittel, herstellt.

10. Verfahren nach mindestens einem der Ansprüche 7 bis 9, dadurch gekennzeichnet, daß man die Katalysatoren in einer Menge im Bereich von 0,02 bis 2 Mol-%, bezogen auf Fettsäuren bzw. Fettsäureester, verwendet.

11. Verfahren nach mindestens einem der Ansprüche 7 bis 10, dadurch gekennzeichnet, daß man als Katalysatoren Rhodiumverbindungen verwendet.

12. Verfahren nach mindestens einem der Ansprüche 1 bis 5, dadurch gekennzeichnet, daß man Rhodiumverbindungen aus der von RhCl₃ und RhBr₃ (einschließlich Hydraten derselben) sowie [(C₂H₄)₂RhCl]₂ gebildeten Gruppe als Katalysatoren verwendet.

## Claims

1. Olefinically unsaturated adducts of propylene with polyunsaturated C₁₈₋₂₂ fatty acids or esters thereof with C₁₋₃₆ alkanols in molar ratios of propylene to the fatty acids or fatty acid esters of 1:1 to 2:1 obtainable by reaction of the fatty acids or fatty acid esters with propylene at elevated temperature and elevated pressure in the presence of compounds of transition metals from the group consisting of Ru, Rh, Pd, Ir and Pt as catalysts.

2. Olefinically unsaturated adducts as claimed in claim 1, characterized in that the polyunsaturated fatty acids contain 2 to 5 and, more particularly, 2 to 3 olefinic double bonds.

3. Olefinically unsaturated adducts as claimed in claim 1 or 2, characterized in that the adducts are produced under a propylene pressure of 5 to 40 bar and at a temperature of 50 to 140°C, optionally in the presence of inert organic solvents.

4. Olefinically unsaturated adducts as claimed in at least one of claims 1 to 3, characterized in that the catalysts are used in a quantity of 0.02 to 2 mol-%, based on fatty acids or fatty acid esters.

5. Olefinically unsaturated adducts as claimed in at least one of claims 1 to 4, characterized in that rhodium compounds are used as catalysts.

6. Olefinically unsaturated adducts as claimed in at least one of claims 1 to 5, characterized in that rhodium compounds from the group consisting of RhCl₃ and RhBr₃ (including hydrates thereof) and [(C₂H₄)₂RhCl]₂ are used as catalysts.

7. A process for the production of olefinically unsaturated adducts of propylene with polyunsaturated fatty acids containing 18 to 22 carbon atoms or esters thereof with C₁₋₃₆ alkanols in molar ratios of propylene to the fatty acids or fatty acid esters of 1:1, characterized in that the fatty acids or fatty acid esters are reacted with propylene at elevated temperature and pressure in the presence of compounds of transition metals from the group consisting of Ru, Rh, Pd, Ir and Pt as catalysts.

8. A process for the production of the olefinically unsaturated adducts as claimed in claim 7, characterized in that polyunsaturated fatty acids containing 2 to 5 and, more particularly, 2 to 3 olefinic double bonds are used.

9. A process as claimed in claim 7 or 8, characterized in that the adducts are produced under a propylene pressure of 5 to 40 bar and at a temperature of 50 to 140°C, optionally in the presence of inert organic solvents.

10. A process as claimed in at least one of claims 7 to 9, characterized in that the catalysts are used in a quantity of 0.02 to 2 mol-%, based on fatty acids or fatty acid esters.

11. A process as claimed in at least one of claims 7 to 10, characterized in that rhodium compounds are used as the catalysts.

12. A process as claimed in at least one of claims 1 to 5, characterized in that rhodium compounds from the group consisting of RhCl₃ and RhBr₃ (including hydrates thereof) and [(C₂H₄)₂RhCl]₂ are used as the catalysts.

## Revendications

1. Adducts oléfiniquement insaturés de propylène sur des acides gras polyinosaturés de 18 à 22 atomes de carbone ou leurs esters d'alcanols en C₁-C₃₆ en rapports nucléaires du propylène aux acides gras ou aux esters d'acides gras dans l'intervalle de 1:1 à 2:1, qu'on peut obtenir par réaction des acides gras ou des esters d'acides gras avec le propylène à haute température et haute pression en présence de composés des métaux de transition du groupe formé par Ru, Rh, Pd, Ir et Pt comme catalyseurs.

2. Adducts oléfiniquement insaturés selon la revendication 1, caractérisés en ce que les acides gras polyinsaturés présentent 2 à 5, notamment 2 à 3 doubles liaisons oléfiniques.

3. Adducts oléfinifiquement insaturés selon la revendication 1 ou 2, caractérisés en ce qu'on prépare les adducts sous une pression de propylène comprise entre 5 et 40 bars et une température de 50 à 140°C, le cas échéant en présence de solvants organiques inertes.

4. Adducts oléfiniquement insaturés selon au moins l'une des revendications 1 à 3, caractérisés en ce qu'on utilise les catalyseurs en une quantité comprise entre 0,02 et 2 % molaire par rapport aux acides gras et aux esters d'acide gras.

5. Adducts oléfininement insaturée selon au moins l'une des revendications 1 à 4, caractérisés en ce que ce qu'on utilise comme catalyseurs des composés de rhodium.

6. Adducts oléfiniment insaturés selon au moins l'une des revendications 1 à 5, caractérisés en ce qu'on utilise comme catalyseurs des composés du rhodium dans le groupe constitué de RhCl₃ et RhB₃ (y compris leurs hydrates) ainsi que de [(C2_{H}4)₂RhCl]₂.

7. Procédé de fabrication d'adducts oléifiniquement insaturés de propylène sur des acides gras polyinsaturés de 18 à 22 atomes de carbone ou leurs esters d'alcanols en C₁-C₃₆ en rapports polaires du propylène aux acides gras ou aux esters d'acides gras dans l'intervalle de 1:1 par réaction des acides gras ou des esters d'acides gras avec le propyléne à haute température et haute pression en présence de composés des métaux de transition du groupe formé par Ru, Rh, Pd, Ir et Pt comme catalyseurs.

8. Procédé de fabrication des adducts oléfiniquement insaturés selon la revendication 7, caractérisé en ce qu'on utilise des acides gras polyinsatirés, qui présentent 2 à 5, de préférence 2 à 3 doubles liaisons oléfinique.

9. Procédé selon les revendications 7 ou 8, caractérisé en ce qu'on prépare les adducts sous une pression de propylène comprise entre 5 et 40 bars et une température de 50 à 140°C, le cas échéant en présence de solvants organiques inertes.

10. Procédé selon au moins une des revendications 7 à 9, caractérisé en ce qu'on utilise les catalyseurs en une quantité comprise entre 0,02 et 2 % molaire par rapport aux acides gras et aux esters d'acide gras.

11. Procédé selon au moins une des revendications 7 à 10, caractérisé en ce que l'on utilise comme catalyseurs des composés du rhodium.

12. Procédé selon au moins une des revendications 1 à 5, caractérisé en ce que l'on utilise des composés du rhodium choisis dans le groupe formé de Cl₃Rh et Br₃Rh (y compris les hydrates de ceux-ci) ainsi que de [(C₂H₄)₂RhCl]₂ comme catalyseurs.
